(19) Europäisches Patentamt
European Patent Office
Office européen des brevets

(11) EP 4 796 568 A1

(12) **EUROPEAN PATENT APPLICATION**
published in accordance with Art. 153(4) EPC

(43) Date of publication:
**26.08.2026 Bulletin 2026/35**

(21) Application number: **24879850.6**

(22) Date of filing: **18.10.2024**

(51) International Patent Classification (IPC):
***C08F 220/10*** (2006.01) ***A61C 7/08*** (2006.01)
***A61C 13/10*** (2006.01) ***A61K 6/15*** (2020.01)
***A61K 6/60*** (2020.01) ***A61K 6/62*** (2020.01)
***A61M 16/06*** (2006.01) ***B29C 64/124*** (2017.01)
***B29C 64/314*** (2017.01) ***B33Y 80/00*** (2015.01)
***C08F 2/44*** (2006.01) ***C08F 290/00*** (2006.01)

(52) Cooperative Patent Classification (CPC):
**A61C 7/08; A61C 13/10; A61K 6/15; A61K 6/60; A61K 6/62; A61M 16/06; B29C 64/124; B29C 64/314; B33Y 80/00; C08F 2/44; C08F 220/10; C08F 290/00**

(86) International application number:
**PCT/JP2024/037281**

(87) International publication number:
**WO 2025/084430 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.10.2023 JP 2023181470**

(71) Applicant: **Kuraray Noritake Dental Inc.**
**Kurashiki-shi, Okayama 710-0801 (JP)**

(72) Inventor: **SUZUKI Kenji**
**Tainai-shi, Niigata 959-2653 (JP)**

(74) Representative: **D Young & Co LLP**
**3 Noble Street**
**London EC2V 7BQ (GB)**

# (54) RESIN COMPOSITION FOR STEREOLITHOGRAPHY

(57) The present invention provides a resin composition for stereolithography that exhibits excellent strength, toughness, water resistance, and stress retention in the shaped article while reducing the amount of leachables. The present invention relates to a resin composition for stereolithography comprising a monofunctional methacrylic polymerizable compound having a plurality of independent aromatic rings (A), a polyfunctional (meth)acrylic polymerizable compound (B), and a photopolymerization initiator (C).

EP 4 796 568 A1

## Description

TECHNICAL FIELD

The present invention relates to a resin composition for stereolithography. More specifically, the invention relates to a stereolithographic material for dental use that exhibits superior strength, toughness, water resistance, and stress retention in the shaped article while achieving excellent biological safety with reduced amounts of leachables from the shaped article. The material is especially suited for applications such as dental occlusal splints, denture base materials, and appliances for the treatment of sleep apnea.

BACKGROUND ART

Various proposals have been made concerning optical 3D fabrication, a method that produces three-dimensional shaped articles through a repeated procedure whereby a liquid photocurable resin is cured into a thin layer under controlled application of necessary amounts of light energy, and another thin layer is cured on the cured layer under controlled application of light after supplying another portion of the liquid photocurable resin onto the previously formed cured layer.

Vat stereolithography is a technique typically used for optical fabrication of three-dimensional shaped articles. In this technique, a liquid photocurable resin composition is placed in a vat, and a computer-controlled ultraviolet laser is selectively applied to the surface of the liquid photocurable resin composition to cure it to a predetermined thickness, forming a cured layer with the desired pattern. Continuously, another cured layer is formed on the cured layer by applying an ultraviolet laser in the same manner to the liquid photocurable resin composition supplied onto the previously cured layer in an amount necessary to form a single layer. This layering process is repeated until it produces the final three-dimensional shaped article. In recent years, this technique has attracted great interest because it enables easy and precision production of the desired three-dimensional shaped article in a relatively short time period, even when the article has a very complex shape.

Three-dimensional shaped articles created through stereolithography are expanding their applications from mere concept models to test models, prototypes, and final products. The field of dental materials is thought to greatly benefit from stereolithography because dental occlusal splints, denture bases, and mouthpiece-like materials used for treating sleep disorders (for example, appliances for preventing bruxism or treating sleep apnea) require shapes that vary from patient to patient, aside from being complex in shape.

Some dental occlusal splints are fitted to adjust tooth alignment or jaw position as in so-called orthodontic mouthpieces or aligners while others are attached to teeth to reduce tooth wear due to clenching. Mouthguards are another type of splints that are worn in the mouth to protect the stomatognathic system and the brain by reducing injuries caused when large external forces are applied to teeth and jawbones during sports activities in contact sports. In orthodontics, the use of these devices has gained wide popularity over the last years due to their favorable aesthetics and convenient removability.

Denture base materials are materials used for the gum as a part of a denture attached to replace missing teeth. The demand for dentures has rapidly increased in recent years because of increasing ageing populations.

Appliances for the treatment of sleep apnea include appliances (oral appliances, or OA) attached to teeth during sleep for the treatment of obstructive sleep apnea syndrome (OSAS), and its use has been rapidly increasing.

Common requirements for dental occlusal splints, denture base materials, and OA include strength, toughness, and water resistance. Low strength creates discomfort by increasing flexure or deformation during wear, whereas a loss of toughness necessitates frequent replacement as it increases susceptibility to breakage from biting forces or deformation during wear. A loss of water resistance causes a reduction of mechanical properties during use, making the appliance practically useless by making the appliance easily deformable or breakable while being worn.

Another consideration is that fabrication of dental occlusal splints, denture base materials, and appliances for the treatment of sleep apnea typically requires taking an impression of the oral cavity. This is seen as a problem because the procedure involves discomfort, and places a burden on patients, in addition to requiring high technical skills.

Recent advances in digital technology have led to approaches that make use of an intraoral optical scan for taking an oral impression, and there have been attempts to apply stereolithography techniques for shaping. For fabrication, a stereolithographic resin composition is used. As a rule, resin compositions that exhibit strength tend to be more brittle, and it has been difficult to provide a cured product having superior strength and toughness.

Against this backdrop, techniques are available that can yield cured products having superior strength and toughness, as described in, for example, Patent Literatures 1 to 5.

Patent Literatures 1 and 2 disclose examples of resin compositions for stereolithography in which a monofunctional (meth)acrylic polymerizable compound having multiple aromatic rings and a specific homopolymer Tg-contained as a polymerizable compound essential to the compositions-is combined with another essential component 2,4,6-trimethyl-

benzoyldiphenylphosphine oxide contained as a photopolymerization initiator.

Patent Literature 3 discloses an example of a resin composition for stereolithography in which a urethane acrylate oligomer and a monofunctional acrylic polymerizable compound having multiple aromatic rings-contained as polymerizable compounds essential to the composition-are combined with another essential component 2,4,6-trimethylbenzoyldiphenylphosphine oxide contained as a photopolymerization initiator.

Patent Literatures 4 and 5 disclose examples in which a specific polyfunctional methacrylate and a monofunctional acrylate having multiple aromatic rings, representing polymerizable compounds contained as essential components, are combined with another essential component 2,4,6-trimethylbenzoyldiphenylphosphine oxide contained as a photopolymerization initiator.

## CITATION LIST

### Patent Literature

Patent Literature 1: WO2020/246610
Patent Literature 2: JP2021-088150 A
Patent Literature 3: WO2019/189566
Patent Literature 4: JP2020-158417 A
Patent Literature 5: WO2021/162007

## SUMMARY OF INVENTION

### Technical Problem

However, examination by the present inventor revealed that Patent Literatures 1 to 5 do not describe anything about stress retention and leachability, though the cured products of the resin compositions for stereolithography are described as having a certain level of strength, toughness, and water resistance. Through further investigation, the present inventor has found areas for improvement in terms of stress retention and leachability.

In particular, it was revealed that, in dental occlusal splints, notably orthodontic aligners, a loss of stress in the aligner diminishes its tooth corrective capability, even if its strength and toughness remain intact.

Additionally, providing toughness, or flexibility, to the cured product of the resin composition for stereolithography alongside strength necessitates changes to the monomer. Because monomers used to impart flexibility possess more flexible molecule structures that permit easier moisture penetration, it leads to a major drawback involving not only reductions in strength, toughness, and stress retention but also increased leaching of degradation products of the photopolymerization initiator or unpolymerized components.

Solving this issue of leaching from the cured product is of great importance particularly in stereolithographic materials, which, compared to non-stereolithographic dental materials, involve an extremely short irradiation time and necessitate the incorporation of a large amount of photopolymerization initiator.

It has been difficult, however, to achieve all of strength, toughness, water resistance, and stress retention along with reduced leaching from the shaped article.

It is accordingly an object of the present invention to provide a resin composition for stereolithography that exhibits excellent strength, toughness, water resistance, and stress retention while reducing leachables.

### Solution to Problem

Specifically, the present invention includes the following.

[1] A resin composition for stereolithography, comprising a monofunctional methacrylic polymerizable compound having a plurality of independent aromatic rings (A), a polyfunctional (meth)acrylic polymerizable compound (B), and a photopolymerization initiator (C).

[2] The resin composition for stereolithography according to [1], wherein the monofunctional methacrylic polymerizable compound having a plurality of independent aromatic rings (A) has two aromatic rings.

[3] The resin composition for stereolithography according to [1] or [2], wherein the monofunctional methacrylic polymerizable compound having a plurality of independent aromatic rings (A) comprises at least one selected from the group consisting of o-phenoxybenzyl methacrylate and m-phenoxybenzyl methacrylate.

[4] The resin composition for stereolithography according to any one of [1] to [3], wherein the polyfunctional (meth)

acrylic polymerizable compound (B) comprises a polyfunctional (meth)acrylic polymerizable compound with a molecular weight of less than 500 (B)-I.

[5] The resin composition for stereolithography according to any one of [1] to [4], wherein the polyfunctional (meth) acrylic polymerizable compound (B) comprises a polyfunctional (meth)acrylic polymerizable compound with a molecular weight of 500 or more (B)-II.

[6] The resin composition for stereolithography according to any one of [1] to [5], wherein the polyfunctional (meth) acrylic polymerizable compound (B) comprises a polyfunctional (meth)acrylic polymerizable compound with a molecular weight of less than 500 (B)-I, and a polyfunctional (meth)acrylic polymerizable compound with a molecular weight of 500 or more (B)-II.

[7] The resin composition for stereolithography according to any one of [1] to [6], wherein the polyfunctional (meth) acrylic polymerizable compound (B) comprises a polyfunctional (meth)acrylic polymerizable compound with a molecular weight of less than 500 and containing a urethane bond (B)-I-1, and/or a (meth)acrylic polymerizable compound with a molecular weight of 500 or more and containing a urethane bond (B)-II-1.

[8] The resin composition for stereolithography according to any one of [1] to [6], wherein the polyfunctional (meth) acrylic polymerizable compound (B) comprises a polyfunctional (meth)acrylic polymerizable compound with a molecular weight of less than 500 and containing a urethane bond (B)-I-1, and a (meth)acrylic polymerizable compound with a molecular weight of 500 or more and containing a urethane bond (B)-II-1.

[9] The resin composition for stereolithography according to [7], which comprises the polyfunctional (meth)acrylic polymerizable compound (B)-I-1, wherein the polyfunctional (meth)acrylic polymerizable compound (B)-I-1 comprises a (meth)acrylate that comprises no polymer structure within a single molecule.

[10] The resin composition for stereolithography according to [7], which comprises the (meth)acrylic polymerizable compound (B)-II-1, wherein the (meth)acrylic polymerizable compound (B)-II-1 comprises a (meth)acrylate that comprises, within a single molecule, at least one structure selected from the group consisting of a polyester, a polycarbonate, a polyurethane, a polyether, a poly-conjugated diene, and a hydrogenated poly-conjugated diene.

[11] The resin composition for stereolithography according to [8] or [10], wherein the (meth)acrylic polymerizable compound (B)-II-1 comprises a (meth)acrylate that comprises, within a single molecule, at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic chain diol unit having a branched structure.

[12] The resin composition for stereolithography according to any one of [1] to [11], wherein the photopolymerization initiator (C) comprises a photopolymerization initiator with a molecular weight of 400 or more (C)-1.

[13] The resin composition for stereolithography according to [12], wherein the photopolymerization initiator with a molecular weight of 400 or more (C)-1 comprises bis(2,4,6-trimethylbenzoyl)phenylphosphine oxide.

[13] A dental material comprising a shaped article of the resin composition for stereolithography of [1] to [13].

[14] A denture base material comprising a shaped article of the resin composition for stereolithography of [1] to [13].

[15] A dental occlusal splint comprising a shaped article of the resin composition for stereolithography of [1] to [13].

[16] A material for treating sleep disorder comprising a shaped article of the resin composition for stereolithography of [1] to [13].

[17] A method for stereolithographically producing a three-dimensional shaped article using the resin composition for stereolithography of [1] to [13].

Advantageous Effects of Invention

A resin composition for stereolithography of the present invention exhibits excellent strength, toughness, water resistance, and stress retention in the shaped article (hereinafter, also referred to as "cured product") while reducing leachables from the shaped article. This makes a resin composition for stereolithography of the present invention suited for dental materials, dental occlusal splints, and denture base materials, as well as materials used for appliances for the treatment of sleep apnea.

In particular, because a resin composition for stereolithography of the present invention can reduce a decrease in stress while maintaining strength and toughness, it can be prevented from losing its tooth corrective capability when used as an orthodontic aligner.

DESCRIPTION OF EMBODIMENTS

The following describes the present invention in detail through embodiments.

In this specification, the upper limits and lower limits of numeric ranges (for example, ranges of contents of components, ranges of values calculated from components, and ranges of physical properties) can be appropriately combined. For example, in this specification, the lower and upper limits of numeric ranges presented in a stepwise fashion

may be combined independently. As an example, in a statement such as "preferably 0.2 to 8.0 mass%, more preferably 0.5 to 5.0 mass%" for the same parameter, it is possible to combine the preferred lower limit (0.2 mass%) with the more preferred upper limit (5.0 mass%) to form a range of "0.2 to 5.0 mass%", or, alternatively, "0.5 to 8.0 mass%."

Concerning numeric ranges, it is also possible to define only the lower limit without specifying the upper limit, for example, such as "12 mass% or more" or "12.5 mass% or more", based on a statement "more preferably 12 to 28 mass%, even more preferably 12.5 to 25 mass%". Similarly, it is possible to define only the upper limit without specifying the lower limit, such as "28 mass% or less" or "25 mass% or less."

Unless specifically indicated otherwise, when the expression of a numeric range is simply denoted as "30 to 70", it indicates 30 or more and 70 or less. Furthermore, for example, when a numeric range is "25 mass% or more and 75 mass% or less", the boundary values of the numeric range may be chosen from any of "more than 25 mass%", "25 mass% or more", "75 mass% or less", and "less than 75 mass%."

Similarly, for example, from statements indicated for the same parameter such as "more preferably 0.05 parts or more by mass, even more preferably 0.1 parts or more by mass" and "more preferably 15 parts or less by mass, even more preferably 10 parts or less by mass", it is possible to combine the more preferred lower limit (0.05 parts or more by mass) with the even more preferred upper limit (10 parts or less by mass) to form the range of "0.05 parts or more by mass and 10 parts or less by mass." Furthermore, as with the foregoing, it is also possible to specify only the lower limit as "0.05 parts or more by mass" or "0.1 parts or more by mass", and, similarly, only the upper limit as "15 parts or less by mass" or "10 parts or less by mass."

The numeric ranges described above are merely examples using mass percentages and parts by mass, and the same applies to various parameters, including molecular weight, flexural strength, and flexural modulus.

The present invention encompasses embodiments combining all or part of the features described in this specification, provided that the present invention can exhibit its effects with such combinations made in various forms within the technical idea of the present invention.

As used herein, the term "polymerizable compound" is used to refer to a polymerizable compound polymerized by the photopolymerization initiator (C) described below.

As used herein, the term "(meth)acryl" is intended to be inclusive of both methacryl and acryl, and the same applies to similar expressions such as "(meth)acrylate", "(meth)acrylic acid ester", "(meth)acrylamide", and "(meth) acryloyloxy."

As used herein, the term "(meth)acrylic polymerizable compound" is intended to be inclusive of both polymerizable compounds having a (meth)acryloxy group as a polymerizable group, and polymerizable compounds having a (meth) acrylamide group as a polymerizable group.

As used herein, "polyfunctional" means that the number of polymerizable groups is more than one.

As used herein, "monofunctional" means having one of the aforementioned polymerizable groups.

As used herein, "molecular weight" refers to a single value calculated from atomic weights when an oligomer or polymer structure is absent. When an oligomer or polymer structure is present, the term "molecular weight" refers to, unless otherwise specified, the weight-average molecular weight determined by gel permeation chromatography (GPC), expressed in terms of polystyrene equivalents.

A resin composition for stereolithography of the present invention comprises a monofunctional methacrylic polymerizable compound having a plurality of independent aromatic rings (A), a polyfunctional (meth)acrylic polymerizable compound (B), and a photopolymerization initiator (C).

[Monofunctional Methacrylic Polymerizable Compound Having a Plurality of Independent Aromatic Rings (A)]

The monofunctional methacrylic polymerizable compound having a plurality of independent aromatic rings (A) (hereinafter, also referred to simply as "monofunctional methacrylic polymerizable compound (A)") reduces the viscosity of the resin composition for stereolithography and improves its shapeability. By the presence of two or more hydrophobic aromatic rings, the monofunctional methacrylic polymerizable compound (A) also has the effect of imparting improved water resistance to the shaped article. By being monofunctional, the monofunctional methacrylic polymerizable compound (A) can also reduce brittleness and provide toughness. Furthermore, the presence of a methacryl group, rather than an acryl group, allows the monofunctional methacrylic polymerizable compound (A) to act more integrally with other components such as polyfunctional (meth)acrylic polymerizable compound (B) in the cured product of the resin composition for stereolithography, reducing the likelihood of impairing stress retention, thereby preventing the resin composition for stereolithography from losing its tooth corrective capability when used as an orthodontic aligner.

Particularly, when the monofunctional methacrylic polymerizable compound (A) is combined with a polyfunctional (meth)acrylic polymerizable compound (B) that comprises a polyfunctional (meth)acrylic polymerizable compound with a molecular weight of less than 500 (B)-I and a polyfunctional (meth)acrylic polymerizable compound with a molecular weight of 500 or more (B)-II (particularly preferably with the combination of polyfunctional (meth)acrylic polymerizable compound (B)-I-1 and polyfunctional (meth)acrylic polymerizable compound (B)-II-1), the resulting cured product can

exhibit excellent strength and stress retention along with improved toughness and water resistance while reducing leachables, making the resin composition for stereolithography suitable for use as a dental occlusal splint (especially, an orthodontic aligner).

In the present invention, it is important that the monofunctional methacrylic polymerizable compound (A) have a plurality of independent aromatic rings. As used herein, "a plurality of independent aromatic rings" excludes structures in which two or more aromatic rings are directly fused together (such as the naphthalene skeleton, phenanthrene skeleton, phenalene skeleton, and anthracene skeleton). By the presence of a plurality of independent aromatic rings, it is possible to achieve low viscosity, improved solubility, and reduced brittleness in the shaped article.

Preferably, the monofunctional methacrylic polymerizable compound (A) does not comprise a skeleton in which two or more aromatic rings are directly fused together.

Examples of structures with a plurality of independent aromatic rings include a biphenyl skeleton, a diphenylmethyl skeleton, a 2,2-diphenylpropane skeleton, a triphenylmethyl skeleton, a diphenylether skeleton, a fluorene skeleton, a carbazole skeleton, and a diphenylamine skeleton. In view of improving solubility and reducing the viscosity of the resin composition for stereolithography and improving the toughness of the cured product, preferred among these are the biphenyl skeleton, diphenylmethyl skeleton, diphenylether skeleton, and diphenylamine skeleton, more preferably the biphenyl skeleton and the diphenylether skeleton, even more preferably the diphenylether skeleton. Here, "skeleton" can be read as referring to a "group."

The multiple independent aromatic rings of the monofunctional methacrylic polymerizable compound (A) may have substituents such as alkyl groups, alkoxy groups, ester groups, acyl groups, alkylamino groups, silyl groups, nitro groups, nitroso groups, and halogen atoms.

The number of substituents is not particularly limited, and is preferably 0 to 6, more preferably 0 to 4, even more preferably 0 to 2, particularly preferably 0 to 1.

Examples of the alkyl groups and the alkyl groups of the alkylamino groups include linear or branched alkyl groups having 1 to 12 carbon atoms, specifically, methyl groups, ethyl groups, n-propyl groups, isopropyl groups, n-butyl groups, isobutyl groups, sec-butyl groups, 2-methylpropyl groups, tert-butyl groups, n-pentyl groups, isopentyl groups, and neopentyl groups.

The alkyl groups and the alkyl groups of the alkylamino groups have preferably 0 to 6 carbon atoms, more preferably 0 to 2 carbon atoms, even more preferably 0 to 1 carbon atom. In view of water resistance, it is preferable not to include substituents having active hydrogens and capable of forming hydrogen bonds, such as hydroxyl groups, acidic groups, amino groups, and urethane groups.

In the present invention, it is important that the monofunctional methacrylic polymerizable compound (A) be monofunctional. The monofunctionality allows the resin composition for stereolithography to have reduced viscosity, reducing the brittleness of the shaped article.

In the present invention, it is particularly important that the monofunctional methacrylic polymerizable compound (A) be a methacrylic polymerizable compound. By being a methacrylic polymerizable compound, the monofunctional methacrylic polymerizable compound (A), together with the polyfunctional (meth)acrylic polymerizable compound (B), can impart stress retention to the shaped article of the resin composition for stereolithography. Acrylic polymerizable compounds tend to impair the stress retention of the shaped article. To describe more specifically, favorable stress retention tends to occur when the polymer microstructure remains intact. In a broad sense, such microstructure can be classified into a physical cohesive structure of the polymer derived primarily from monofunctional methacrylic polymerizable compound (A), and a covalent crosslinked structure formed by the polyfunctional (meth)acrylic polymerizable compound. Presumably, the retention of these two structures contributes to stress retention. Because polymers of methacrylic polymerizable compounds have higher glass transition temperatures than polymers of acrylic polymerizable compounds, the use of monofunctional methacrylic polymerizable compound (A) leads to a stronger physical cohesive structure.

Examples of the monofunctional methacrylic polymerizable compound (A) include (meth)acrylic acid ester compounds having two or more aromatic rings, such as o-phenylphenyl methacrylate, m-phenylphenyl methacrylate, p-phenylphenyl methacrylate, ethoxylated o-phenylphenol methacrylate, ethoxylated m-phenylphenol methacrylate, ethoxylated p-phenylphenol methacrylate, propoxylated o-phenylphenol methacrylate, propoxylated m-phenylphenol methacrylate, propoxylated p-phenylphenol methacrylate, butoxylated o-phenylphenol methacrylate, butoxylated m-phenylphenol methacrylate, butoxylated p-phenylphenol methacrylate, diphenylmethyl methacrylate, 4-(1-methyl-1-phenylethyl)methacrylate, triphenylmethyl methacrylate, o-phenoxyphenyl methacrylate, m-phenoxyphenyl methacrylate, p-phenoxyphenyl methacrylate, o-phenoxybenzyl methacrylate, m-phenoxybenzyl methacrylate, p-phenoxybenzyl methacrylate, 2-(o-phenoxyphenyl)ethyl methacrylate, 2-(m-phenoxyphenyl)ethyl methacrylate, 2-(p-phenoxyphenyl)ethyl methacrylate, 3-(o-phenoxyphenyl)propyl methacrylate, 3-(m-phenoxyphenyl)propyl methacrylate, 3-(p-phenoxyphenyl)propyl methacrylate, 9-methacryloyloxyfluorene, 9-methacryloyloxymethylfluorene, N-methacryloylcarbazole, and N-methacryloylmethylcarbazole. The monofunctional methacrylic polymerizable compound (A) may be used alone, or two or more thereof may be used in combination. In view of reducing the viscosity of the resin composition for

stereolithography and improving the stress retention of the shaped article in combination with polyfunctional (meth)acrylic polymerizable compound (B), more preferred among these are ethoxylated o-phenylphenol methacrylate, ethoxylated m-phenylphenol methacrylate, ethoxylated p-phenylphenol methacrylate, o-phenoxybenzyl methacrylate, m-phenoxybenzyl methacrylate, p-phenoxybenzyl methacrylate, 2-(o-phenoxyphenyl)ethyl methacrylate, 2-(m-phenoxyphenyl)ethyl methacrylate, and 2-(p-phenoxyphenyl)ethyl methacrylate, even more preferably ethoxylated o-phenylphenol methacrylate, ethoxylated m-phenylphenol methacrylate, ethoxylated p-phenylphenol methacrylate, o-phenoxybenzyl methacrylate, m-phenoxybenzyl methacrylate, and p-phenoxybenzyl methacrylate, particularly preferably ethoxylated o-phenylphenol methacrylate, o-phenoxybenzyl methacrylate, and m-phenoxybenzyl methacrylate, most preferably m-phenoxybenzyl methacrylate.

The content of the monofunctional methacrylic polymerizable compound (A) in a resin composition for stereolithography of the present invention is preferably 1.0 to 60 mass% in total 100 mass% of the monofunctional methacrylic polymerizable compound (A), the polyfunctional (meth)acrylic polymerizable compound (B), and other polymerizable compounds (hereinafter, these will be also referred to collectively as "polymerizable compounds"). In view of even superior shapeability and providing even greater toughness, water resistance, and stress retention in the cured product, the content of monofunctional methacrylic polymerizable compound (A) is more preferably 2.5 to 50 mass%, even more preferably 5 to 45 mass%, particularly preferably 6 to 40 mass%.

In view of even superior shapeability and providing even greater toughness, water resistance, and stress retention in the cured product, the content of monofunctional methacrylic polymerizable compound (A) in total 100 mass% of the resin composition for stereolithography is preferably 1.0 to 60 mass%, more preferably 2.5 to 50 mass%, even more preferably 5 to 45 mass%, particularly preferably 6 to 40 mass%.

[Polyfunctional (Meth)Acrylic Polymerizable Compound (B)]

In a resin composition for stereolithography of the present invention, the polyfunctional (meth)acrylic polymerizable compound (B) is used to impart curability to the resin composition for stereolithography and strength and toughness to the cured product, in combination with the photopolymerization initiator (C).

The polyfunctional (meth)acrylic polymerizable compound (B) also acts integrally with other components such as monofunctional methacrylic polymerizable compound (A) in the cured product of the resin composition for stereolithography, reducing the likelihood of impairing stress retention, thereby preventing the resin composition for stereolithography from losing its tooth corrective capability when used as an orthodontic aligner. The polyfunctional (meth)acrylic polymerizable compound (B) may be used alone, or two or more thereof may be used in combination.

Preferably, the polyfunctional (meth)acrylic polymerizable compound (B) comprises a polyfunctional (meth) acrylic polymerizable compound with a molecular weight of less than 500 (B)-I (hereinafter, also referred to simply as "polyfunctional (meth)acrylic polymerizable compound (B)-I"), and/or a polyfunctional (meth)acrylic polymerizable compound with a molecular weight of 500 or more (B)-II (hereinafter, also referred to simply as "polyfunctional (meth) acrylic polymerizable compound (B)-II"). The polyfunctional (meth)acrylic polymerizable compound (B)-I is suitably used to impart especially strength to the cured product of the resin composition for stereolithography, whereas the polyfunctional (meth)acrylic polymerizable compound (B)-II is suitably used to impart especially toughness to the cured product of the resin composition for stereolithography.

In view of superior water resistance, the polyfunctional (meth)acrylic polymerizable compound with a molecular weight of less than 500 (B)-I is preferably one that does not contain a polymer structure within a single molecule, more preferably a methacrylate that does not contain a polymer structure within a single molecule. The absence of a polymer structure leads to reduced content of repeating units of polar functional groups per molecule, which tends to improve water resistance.

Examples of the polymer structure include polyester, polycarbonate, polyurethane, polyether, polyamide, polyimide, polyarylate, and polyacrylate.

Examples of the polyfunctional (meth)acrylic polymerizable compound (B)-I include a polyfunctional (meth) acrylic polymerizable compound containing a urethane bond (B)-I-1 (hereinafter, also referred to simply as "polyfunctional (meth)acrylic polymerizable compound (B)-I-1"), and a polyfunctional (meth)acrylic polymerizable compound containing no urethane bond (B)-I-2 (hereinafter, also referred to simply as "polyfunctional (meth)acrylic polymerizable compound (B)-I-2"). In view of even superior toughness of the cured product and allowing the urethane bond to also serve as a hydrogen donating structure and achieve even greater curability and a further reduction of leachables from the cured product, as well as achieving even greater water resistance and stress retention, it is preferable to comprise a polyfunctional (meth)acrylic polymerizable compound (B)-I-1. More preferably, the polyfunctional (meth)acrylic polymerizable compound (B)-I-1 is a (meth)acrylate that does not contain a polymer structure within a single molecule.

As an example, the polyfunctional (meth)acrylic polymerizable compound (B)-I-1 can be synthesized with ease by an addition reaction of an isocyanate group-containing compound having an alkylene backbone or phenylene backbone with a (meth)acrylic compound having a hydroxyl group (-OH).

The compound having an isocyanate group, and the (meth)acrylic compound having a hydroxyl group (-OH) may be compounds identical or similar to those exemplified below in the production of polyfunctional urethanized (meth)acrylic polymerizable compound (B)-II-1.

The addition reaction may be performed using known methods and conditions, and is not particularly limited.

Examples of the polyfunctional (meth)acrylic polymerizable compound (B)-I-1 include polyfunctional urethanized (meth)acrylates such as hexamethylenebis(2-carbamoyloxyethyl)dimethacrylate, 2,2,4-trimethylhexamethylenebis(2-carbamoyloxyethyl)dimethacrylate (commonly known as UDMA), 2,4-tolylenebis(2-carbamoyloxyethyl)di(meth)acrylate, bishydroxyethyl methacrylate-isophorone diurethane, and 2,4-tolylenebis(2-carbamoyloxyethyl)dimethacrylate. These may be used alone, or two or more thereof may be used in combination. In view of the strength and toughness of the shaped article, preferred among these are 2,2,4-trimethylhexamethylenebis(2-carbamoyloxyethyl)dimethacrylate, more preferably 2,2,4-trimethylhexamethylenebis(2-carbamoyloxyethyl)dimethacrylate, and bishydroxyethyl methacrylate-isophorone diurethane, even more preferably 2,2,4-trimethylhexamethylenebis(2-carbamoyloxyethyl)dimethacrylate.

In view of providing even superior strength, water resistance, and stress retention in the shaped article when combined with other components, the content of the polyfunctional (meth)acrylic polymerizable compound (B)-I is preferably 10 to 90 mass%, more preferably 15 to 80 mass%, even more preferably 20 to 70 mass%, particularly preferably 30 to 60 mass% in total 100 mass% of the polymerizable compounds.

In another certain preferred embodiment, in view of providing even superior strength and stress retention in the shaped article, the content of the polyfunctional (meth)acrylic polymerizable compound (B)-I is preferably 20 to 80 mass%, more preferably 30 to 75 mass%, even more preferably 35 to 75 mass%, particularly preferably 40 to 70 mass% in total 100 mass% of the polyfunctional (meth)acrylic polymerizable compound (B).

In yet another certain preferred embodiment, in view of providing even superior strength, toughness, water resistance, and stress retention in the shaped article when combined with other components, the content of the polyfunctional (meth)acrylic polymerizable compound (B)-I is preferably 10 to 70 mass%, more preferably 15 to 68 mass%, even more preferably 20 to 65 mass%, particularly preferably 30 to 60 mass% in total 100 mass% of the resin composition for stereolithography.

In any of the embodiments above, in view of enabling a further reduction of leachables from the cured product, the content of polyfunctional (meth)acrylic polymerizable compound (B)-I can be read as referring to the content of polyfunctional (meth)acrylic polymerizable compound (B)-I-1.

In another certain preferred embodiment, in view of providing even superior strength and stress retention of the shaped article and allowing the urethane bond to also serve as a hydrogen donating structure and achieve even greater curability and a further reduction of leachables from the cured product, the content of the polyfunctional (meth)acrylic polymerizable compound (B)-I-1 in the polyfunctional (meth)acrylic polymerizable compound (B)-I is preferably 20 mass% or more, more preferably 40 mass% or more, even more preferably 60 mass% or more, particularly preferably 80 mass% or more in total 100 mass% of the polyfunctional (meth)acrylic polymerizable compound (B)-I.

In certain preferred embodiments, the content of the polyfunctional (meth)acrylic polymerizable compound (B)-I-1 in the polyfunctional (meth)acrylic polymerizable compound (B)-I may be 100 mass%.

Examples of the polyfunctional (meth)acrylic polymerizable compound (B)-I include bifunctional (meth)acrylic polymerizable compounds, and tri- and higher-functional (meth)acrylic polymerizable compounds. However, in view of toughness of the cured product, preferred are bifunctional (meth)acrylic polymerizable compounds.

Examples of the polyfunctional (meth)acrylic polymerizable compound (B)-I-2 include 2,2-bis((meth)acryloyloxyphenyl)propane, 2,2-bis(4-(meth)acryloyloxyethoxyphenyl)propane, 2,2-bis(4-(meth)acryloyloxydiethoxyphenyl)propane, 2,2-bis(4-(meth)acryloyloxypropoxyphenyl)propane, 2,2-bis(4-(meth)acryloyloxytetraethoxyphenyl)propane, 2,2-bis(4-(meth)acryloyloxypentaethoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxyisopropoxyphenyl)propane, 2,2-bis(4-(meth)acryloyloxypolyethoxyphenyl)propane (for example, with an average of 2.6 moles of ethoxy group added), 1,4-bis(2-(meth)acryloyloxyethyl)pyromellitate, glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, and tricyclodecane dimethanol di(meth)acrylate.

Examples of the tri- and higher-functional polymerizable compounds include trimethylolpropane tri(meth)acrylate, trimethylolethane tri(meth)acrylate, trimethylol methane tri(meth)acrylate, pentaerythritol tri(meth)acrylate, pentaerythritol tetra(meth)acrylate, dipentaerythritol penta(meth)acrylate, and 1,7-di(meth)acryloyloxy-2,2,6,6-tetra(meth)acryloyloxymethyl-4-oxaheptane.

The polyfunctional (meth)acrylic polymerizable compound (B)-I requires a molecular weight of less than 500. In

view of strength and toughness, the preferred molecular weight is 250 or more and 495 or less, more preferably 300 or more and 490 or less.

In a resin composition for stereolithography of the present invention, the polyfunctional (meth)acrylic polymerizable compound with a molecular weight of 500 or more (B)-II is suitably used to impart toughness to the cured product.

The polyfunctional (meth)acrylic polymerizable compound (B)-II can be classified into a polyfunctional (meth) acrylic polymerizable compound that contains an oligomer or polymer structure, and a polyfunctional (meth)acrylic polymerizable compound that does not contain an oligomer or polymer structure. However, in view of providing superior strength and toughness in the shaped article when combined with other components, it is preferable to comprise a polyfunctional (meth)acrylic polymerizable compound that contains an oligomer or polymer structure.

The polyfunctional (meth)acrylic polymerizable compound (B)-II includes a polyfunctional (meth)acrylic polymerizable compound containing a urethane bond (B)-II-1 (hereinafter, also referred to simply as "polyfunctional (meth) acrylic polymerizable compound (B)-II-1"), and a polyfunctional (meth)acrylic polymerizable compound containing no urethane bond (B)-II-2. However, in view of superior toughness of the cured product and allowing the urethane bond to also serve as a hydrogen donating structure and achieve even greater curability and a further reduction of leachables from the cured product, it is preferable to comprise a polyfunctional (meth)acrylic polymerizable compound (B)-II-1, more preferably a polyfunctional urethanized (meth)acrylic polymerizable compound (B)-II-1 that contains an oligomer or polymer structure.

In view of even superior toughness of the cured product and allowing the urethane bond to also serve as a hydrogen donating structure and achieve even greater curability and a further reduction of leachables from the cured product, a certain preferred embodiment is, for example, a resin composition for stereolithography that comprises a polyfunctional (meth)acrylic polymerizable compound (B)-I-1, and/or a polyfunctional (meth)acrylic polymerizable compound (B)-II-1.

In view of superior strength and stress retention of the cured product and providing even greater toughness and water resistance in the cured product while reducing leachables from the cured product, a particularly preferred embodiment is, for example, a resin composition for stereolithography that comprises a polyfunctional (meth)acrylic polymerizable compound (B)-I-1, and a polyfunctional (meth)acrylic polymerizable compound (B)-II-1.

When the polyfunctional (meth)acrylic polymerizable compound (B)-II-1 contains an oligomer or polymer structure, the polyfunctional (meth)acrylic polymerizable compound (B)-II-1 containing an oligomer or polymer structure can be easily synthesized through an addition reaction between a polyol having a polymer backbone (e.g., a polymer structure such as a polyester, a polycarbonate, a polyurethane, a polyether, a polydiene, and a hydrogenated polydiene), a compound having an isocyanate group (-NCO), and a (meth)acrylic compound having a hydroxyl group (-OH). The polyfunctional (meth)acrylic polymerizable compound (B)-II-1 containing an oligomer or polymer structure can also be easily synthesized by allowing lactone or alkylene oxide to undergo a ring-opening addition reaction with a (meth)acrylic compound having a hydroxyl group, and causing the resulting compound with a terminal hydroxyl group to undergo an addition reaction with a compound having an isocyanate group.

When the polyfunctional (meth)acrylic polymerizable compound (B)-II-1 contains an oligomer or polymer structure, the polyfunctional (meth)acrylic polymerizable compound (B)-II-1 containing an oligomer or polymer structure is preferably a (meth)acrylate that comprises, within a single molecule, at least one structure selected from the group consisting of a polyester, a polycarbonate, a polyurethane, a polyether, a poly-conjugated diene, and a hydrogenated poly-conjugated diene.

In these structures, examples of the polyester include copolymers of dicarboxylic acids (for example, aromatic dicarboxylic acids such as phthalic acid and isophthalic acid, and unsaturated aliphatic dicarboxylic acids such as maleic acid) and aliphatic diols having 2 to 18 carbon atoms, copolymers of dicarboxylic acids (for example, saturated aliphatic dicarboxylic acids such as adipic acid and sebacic acid) and aliphatic diols having 2 to 18 carbon atoms, β-propiolactone polymers, γ-butyrolactone polymers, δ-valerolactone polymers, ε-caprolactone polymers, and copolymers of these. Preferred are copolymers of dicarboxylic acids (preferably, aromatic dicarboxylic acids, and unsaturated aliphatic dicarboxylic acids) and aliphatic diols having 2 to 12 carbon atoms, and copolymers of dicarboxylic acids (preferably, saturated aliphatic dicarboxylic acids) and aliphatic glycols having 2 to 12 carbon atoms.

Examples of the polycarbonate include polycarbonates derived from C2 to C18 aliphatic diols, polycarbonates derived from bisphenol A, and polycarbonates derived from C2 to C18 aliphatic diols and bisphenol A. Preferred are polycarbonates derived from C2 to C12 aliphatic diols, polycarbonates derived from bisphenol A, and polycarbonates derived from C2 to C12 aliphatic diols and bisphenol A.

Examples of the polyurethane include polymers of C2 to C18 aliphatic diols and C1 to C18 diisocyanates. Preferred are polymers of C2 to C12 aliphatic diols and C1 to C12 diisocyanates.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarne-

sene, and hydrogenated products of these.

In view of superior toughness and water resistance, preferred among these are the polyester structures.

When the polyfunctional (meth)acrylic polymerizable compound (B)-II-1 contains an oligomer or polymer structure, it is preferable in view of even superior toughness and water resistance that the polyfunctional (meth)acrylic polymerizable compound (B)-II-1 containing an oligomer or polymer structure is a (meth)acrylate that comprises, within a single molecule, at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic chain diol unit having a branched structure.

Examples of the polyester include copolymers with a structure derived from a C4 to C18 aliphatic chain diol unit having a branched structure, and with a structure derived from a C4 to C18 aliphatic chain dicarboxylic acid and/or aromatic dicarboxylic acid unit having no branched structure.

Examples of the polycarbonate include copolymers with a structure derived from a C4 to C18 aliphatic chain diol unit having a branched structure, and with a structure derived from a C4 to C18 aliphatic chain diol unit having no branched structure.

Examples of the polyurethane include structures derived from a C4 to C18 aliphatic chain diol unit having a branched structure, and polycondensates of diisocyanate compounds.

Examples of the polyether include polyethers with a structure derived from a C4 to C18 aliphatic chain diol unit having a branched structure, and polyethers with a structure derived from a C4 to C18 aliphatic chain diol unit having no branched structure.

Examples of the poly-conjugated diene include homopolymers or copolymers of conjugated diene polymerizable compounds. Examples of the conjugated diene polymerizable compounds include 1,3-butadiene, isoprene, 2,3-dimethyl-1,3-butadiene, 1,3-pentadiene, and 1,3-hexadiene.

Examples of the hydrogenated poly-conjugated diene include hydrogenated polybutadiene, hydrogenated polyisoprene, and hydrogenated polyisobutylene.

Among these, in view of superior toughness and water resistance, it is preferable to comprise, as a polymer backbone, at least one structure selected from the group consisting of a polyester, a polycarbonate, a polyether, and a hydrogenated poly-conjugated diene, more preferably at least one structure selected from the group consisting of a polyester and a polycarbonate, even more preferably a polyester.

Examples of the diols constituting the C4 to C18 aliphatic chain diol unit having a branched structure include 2-methyl-1,3-propanediol, 2,2-diethyl-1,3-propanediol, 1,3-butanediol, 2-methyl-1,4-butanediol, neopentyl glycol, 3-methyl-1,5-pentanediol, 2-methyl-1,8-octanediol, 2,7-dimethyl-1,8-octanediol, 2-methyl-1,9-nonanediol, 2,8-dimethyl-1,9-nonanediol, 2-methyl-1,10-decanediol, 2,9-dimethyl-1,10-decanediol, 2-methyl-1,11-undecanediol, 2,10-dimethyl-1,11-undecanediol, 2-methyl-1,12-dodecanediol, 2,11-dimethyl-1,12-dodecanediol, 2-methyl-1,13-tridecanediol, 2,12-dimethyl-1,13-tridecanediol, 2-methyl-1,14-tetradecanediol, 2,13-dimethyl-1,14-tetradecanediol, 2-methyl-1,15-pentadecanediol, 2,14-dimethyl-1,15-pentadecanediol, 2-methyl-1,16-hexadecanediol, and 2,15-dimethyl-1,16-hexadecanediol. In view of providing a resin composition for stereolithography having superior curability and low viscosity, preferred for use as polyols are C5 to C12 aliphatic diols having a methyl-group side chain, such as 2-methyl-1,4-butanediol, 3-methyl-1,5-pentanediol, 2-methyl-1,8-octanediol, 2,7-dimethyl-1,8-octanediol, 2-methyl-1,9-nonanediol, and 2,8-dimethyl-1,9-nonanediol, more preferably 2-methyl-1,4-butanediol, 3-methyl-1,5-pentanediol, 2-methyl-1,8-octanediol, and 2,7-dimethyl-1,8-octanediol, even more preferably 3-methyl-1,5-pentanediol, and 2-methyl-1,8-octanediol.

Examples of the compound having an isocyanate group include hexamethylene diisocyanate (HDI), tolylene diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

Examples of the (meth)acrylic compound having a hydroxyl group include hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-(2-hydroxy-3-(meth)acryloyloxypropoxy)phenyl]propane, 1,2-bis[3-(meth)acryloyloxy-2-hydroxypropoxy]ethane, pentaerythritol tri(meth)acrylate, and tri or tetra(meth)acrylates of dipentaerythritol; and hydroxy(meth)acrylamide compounds such as N-hydroxyethyl(meth)acrylamide, and N,N-bis(2-hydroxyethyl)(meth)acrylamide.

When the desired polyfunctional (meth)acrylic polymerizable compound (B)-II-1 is a (meth)acrylate compound, it can be produced by selecting a hydroxy(meth)acrylate compound.

The addition reaction between a compound having an isocyanate group and a (meth)acrylic compound having a hydroxyl group may be performed using known methods and conditions, and is not particularly limited.

Examples of the polyfunctional urethanized (meth)acrylic polymerizable compound (B)-II-1 with no polymer structure include dipentaerythritol penta(meth)acrylate hexamethylene diisocyanate urethane prepolymer.

The polyfunctional (meth)acrylic polymerizable compound (B)-II-1 requires a molecular weight of 500 or more. The molecular weight of polyfunctional (meth)acrylic polymerizable compound (B)-II-1 is preferably 600 or more, more preferably 700 or more. In view of strength and toughness, the molecular weight is even more preferably 750 or more, particularly preferably 1,000 or more. The molecular weight of polyfunctional (meth)acrylic polymerizable compound (B)-II-1 is preferably 6,000 or less, more preferably 5,500 or less. In view of strength and toughness, the molecular weight is even more preferably 5,000 or less, particularly preferably 3,000 or less.

Taken together, in view of strength and toughness, the preferred molecular weight of polyfunctional (meth)acrylic polymerizable compound (B)-II-1 is 750 to 5,000, more preferably 1,000 to 3,000.

Examples of the polyfunctional (meth)acrylic polymerizable compound (B)-II include bifunctional (meth)acrylic polymerizable compounds, and tri- and higher-functional (meth)acrylic polymerizable compounds.

In view of providing even superior strength, toughness, water resistance, and stress retention in the shaped article when combined with other components such as monofunctional methacrylic polymerizable compound (A), a certain preferred embodiment is, for example, a resin composition for stereolithography in which the polyfunctional (meth)acrylic polymerizable compound (B) comprises a polyfunctional (meth)acrylic polymerizable compound with a molecular weight of less than 500 (B)-I, and a polyfunctional (meth)acrylic polymerizable compound with a molecular weight of 500 or more (B)-II.

In view of providing even superior strength, toughness, water resistance, and stress retention in the shaped article when combined with other components such as monofunctional methacrylic polymerizable compound (A), another certain preferred embodiment is, for example, a resin composition for stereolithography in which the polyfunctional (meth)acrylic polymerizable compound (B) comprises a polyfunctional (meth)acrylic polymerizable compound with a molecular weight of less than 500 and containing a urethane bond (B)-I-1, and/or a (meth)acrylic polymerizable compound with a molecular weight of 500 or more and containing a urethane bond (B)-II-1.

In a certain preferred embodiment, in view of even superior strength, toughness, water resistance, and stress retention of the shaped article and allowing the urethane bond to also serve as a hydrogen donating structure and achieve even greater curability and a further reduction of leachables from the cured product when combined with other components, the content of polyfunctional (meth)acrylic polymerizable compound (B)-II-1 is preferably 5 to 80 mass%, more preferably 10 to 70 mass%, even more preferably 15 to 60 mass%, particularly preferably 20 to 50 mass% in total 100 mass% of the polymerizable compounds.

In another certain preferred embodiment, in view of even superior strength, toughness, water resistance, and stress retention of the shaped article and allowing the urethane bond to also serve as a hydrogen donating structure and achieve even greater curability and a further reduction of leachables from the cured product when combined with other components, the content of polyfunctional (meth)acrylic polymerizable compound (B)-II-1 is preferably 10 to 75 mass%, more preferably 15 to 70 mass%, even more preferably 20 to 65 mass%, particularly preferably 25 to 60 mass% in total 100 mass% of the polyfunctional (meth)acrylic polymerizable compound (B).

In yet another certain preferred embodiment, in view of even superior strength, toughness, water resistance, and stress retention of the shaped article and allowing the urethane bond to also serve as a hydrogen donating structure and achieve even greater curability and a further reduction of leachables from the cured product when combined with other components, the content of polyfunctional (meth)acrylic polymerizable compound (B)-II-1 is preferably 1 to 70 mass%, more preferably 5 to 60 mass%, even more preferably 10 to 50 mass%, particularly preferably 20 to 40 mass% in total 100 mass% of the resin composition for stereolithography.

In view of even superior strength, toughness, water resistance, and stress retention of the cured product and allowing the urethane bond to also serve as a hydrogen donating structure and achieve even greater curability and a further reduction of leachables from the cured product when combined with other components, the content of polyfunctional (meth)acrylic polymerizable compound (B)-II-1 is preferably 20 mass% or more, more preferably 40 mass% or more, even more preferably 60 mass% or more, particularly preferably 80 mass% or more in total 100 mass% of the polyfunctional (meth)acrylic polymerizable compound (B)-II.

The content of the polyfunctional (meth)acrylic polymerizable compound (B)-II-1 in the polyfunctional (meth) acrylic polymerizable compound (B)-II may be 100 mass%.

Examples of the polyfunctional (meth)acrylic polymerizable compound containing no urethane bond (B)-II-2 include bifunctional (meth)acrylic polymerizable compounds, and tri- and higher-functional (meth)acrylic polymerizable compounds.

Examples of the polyfunctional (meth)acrylic polymerizable compound containing no urethane bond (B)-II-2 include 2,2-bis[4-(2-hydroxy-3-methacryloyloxypropoxy)phenyl]propane (commonly known as Bis-GMA), 2,2-bis(4-(meth)acryloyloxypolyethoxyphenyl)propane (for example, with an average of 25 or more moles of ethoxy group added), and polyethylene glycol di(meth)acrylate.

In view of even superior water resistance, the polyfunctional (meth)acrylic polymerizable compound containing no urethane bond (B)-II-2 is preferably a polymerizable compound with no hydroxyl group, for example.

In view of providing even superior strength, toughness, water resistance, and stress retention in the cured

product when combined with other components, the content of the polyfunctional (meth)acrylic polymerizable compound (B)-II in a resin composition for stereolithography of the present invention is preferably 1 to 80 mass%, more preferably 5 to 70 mass%, even more preferably 10 to 60 mass%, particularly preferably 20 to 50 mass% in total 100 mass% of the polymerizable compounds. The polyfunctional (meth)acrylic polymerizable compound (B)-II may be used alone, or two or more thereof may be used in combination.

In view of providing even superior strength, toughness, water resistance, and stress retention in the cured product when combined with other components, the content of the polyfunctional (meth)acrylic polymerizable compound (B)-II in a resin composition for stereolithography of the present invention is preferably 10 to 60 mass%, more preferably 15 to 59 mass%, even more preferably 20 to 50 mass%, particularly preferably 25 to 45 mass% in total 100 mass% of the resin composition for stereolithography.

In view of even superior strength, toughness, water resistance, and stress retention of the cured product, the mass ratio of the content of the polyfunctional (meth)acrylic polymerizable compound (B)-I and the content of the polyfunctional (meth)acrylic polymerizable compound (B)-II is preferably 5:95 to 95:5, more preferably 10:90 to 90:10, even more preferably 20:80 to 80:20 in a resin composition for stereolithography in which the polyfunctional (meth)acrylic polymerizable compound (B) comprises a polyfunctional (meth)acrylic polymerizable compound with a molecular weight of less than 500 (B)-I, and a polyfunctional (meth)acrylic polymerizable compound with a molecular weight of 500 or more (B)-II.

In view of providing particularly superior strength and water resistance in the cured product, a certain preferred embodiment is, for example, a resin composition for stereolithography in which the polyfunctional (meth)acrylic polymerizable compound (B) comprises a polyfunctional (meth)acrylic polymerizable compound with a molecular weight of less than 500 (B)-I, and a polyfunctional (meth)acrylic polymerizable compound with a molecular weight of 500 or more (B)-II, and the mass ratio (B)-I:(B)-II of the content of the polyfunctional (meth)acrylic polymerizable compound (B)-I and the content of the polyfunctional (meth)acrylic polymerizable compound (B)-II is 51:49 to 95:5. The mass ratio (B)-I:(B)-II is more preferably 52:48 to 90:10, even more preferably 55:45 to 80:20.

The content of the polyfunctional (meth)acrylic polymerizable compound (B) in a resin composition for stereolithography of the present invention is preferably 10 to 95 mass% in total 100 mass% of the polymerizable compounds. In view of providing even superior strength, toughness, water resistance, and stress retention in the shaped article when combined with other components, the content of the polyfunctional (meth)acrylic polymerizable compound (B) is more preferably 20 to 92 mass%, even more preferably 30 to 90 mass%, particularly preferably 40 to 85 mass%. The polyfunctional (meth)acrylic polymerizable compound (B) may be used alone, or two or more thereof may be used in combination.

In view of providing even superior strength, toughness, water resistance, and stress retention in the shaped article when combined with other components, the content of the polyfunctional (meth)acrylic polymerizable compound (B) in a resin composition for stereolithography of the present invention is preferably 10 to 96 mass%, more preferably 20 to 94 mass%, even more preferably 30 to 92 mass%, particularly preferably 40 to 90 mass% in total 100 mass% of the resin composition for stereolithography.

In view of providing even superior strength, toughness, water resistance, and stress retention in the shaped article by the combination of monofunctional methacrylic polymerizable compound (A) and polyfunctional (meth)acrylic polymerizable compound (B), the mass ratio (A):(B) of the content of the monofunctional methacrylic polymerizable compound (A) and the content of the polyfunctional (meth)acrylic polymerizable compound (B) in a resin composition for stereolithography of the present invention is preferably 1:1 to 1:10, more preferably 1:1.1 to 1:8, even more preferably 1:1.2 to 1:8, particularly preferably 1:1.7 to 1:5.

In a certain preferred embodiment, in view of providing even superior strength, toughness, water resistance, and stress retention in the shaped article by the combination of monofunctional methacrylic polymerizable compound (A) and polyfunctional (meth)acrylic polymerizable compound (B)-I, the mass ratio (A):(B)-I of the content of the monofunctional methacrylic polymerizable compound (A) and the content of the polyfunctional (meth)acrylic polymerizable compound (B)-I is preferably 1:1 to 1:10, more preferably 1:1.1 to 1:8, even more preferably 1:1.2 to 1:8, particularly preferably 1:1.7 to 1:5.

In all of the embodiments described in this specification, the content of polyfunctional (meth)acrylic polymerizable compound (B)-II can be read as referring to the content of the (meth)acrylic polymerizable compound with a molecular weight of 500 or more and containing a urethane bond (B)-II-1, or the content of the polyfunctional (meth)acrylic polymerizable compound containing no urethane bond (B)-II-2, unless specifically indicated otherwise.

Without departing from the spirit of the present invention, a resin composition for stereolithography of the present invention may comprise monofunctional (meth)acrylic polymerizable compounds other than the monofunctional methacrylic polymerizable compound (A), in order to adjust the viscosity of the resin composition for stereolithography or the mechanical properties of the shaped article.

Examples of monofunctional (meth)acrylic polymerizable compounds other than the monofunctional methacrylic polymerizable compound (A) include monofunctional acrylic polymerizable compounds having a plurality of independent

aromatic rings, alicyclic (meth)acrylic acid ester compounds, chain (meth)acrylic acid ester compounds, nitrogen atom-containing cyclic (meth)acrylic acid ester compounds, and cyclic (meth)acrylamide compounds. Examples are (meth) acrylic acid ester compounds having a single aromatic ring, such as phenyl (meth)acrylate, benzyl (meth)acrylate, phenoxyethyl (meth)acrylate, phenoxybutyl (meth)acrylate, phenoxydiethylene glycol (meth)acrylate, phenoxypolyethylene glycol (meth)acrylate, 4-methylphenyl (meth)acrylate, 4-n-butylphenyl (meth)acrylate, 4-t-butylphenyl (meth)acrylate, 4-nonylphenyl (meth)acrylate, o-2-propenylphenyl (meth)acrylate, benzhydrol (meth)acrylate, and cumylphenol (meth)acrylate; alicyclic (meth)acrylic acid ester compounds such as 2-(1-adamantyl)propyl (meth)acrylate, 2-methyladamantan-2-yl (meth)acrylate, 2-ethyladamantan-2-yl (meth)acrylate, 2-n-propyladamantan-2-yl (meth)acrylate, 2-isopropyladamantan-2-yl (meth)acrylate, 1-(adamantan-1-yl)-1-methylethyl (meth)acrylate, 1-(adamantan-1-yl)-1-ethylethyl (meth)acrylate, 1-(adamantan-1-yl)-1-methylpropyl (meth)acrylate, and 1-(adamantan-1-yl)-1-ethylpropyl (meth) acrylate; nitrogen atom-containing cyclic (meth)acrylic acid ester compounds such as pentamethylpiperidinyl (meth) acrylate, tetramethylpiperidinyl (meth)acrylate, and 4-(pyrimidin-2-yl)piperazin-1-yl (meth)acrylate; and cyclic (meth) acrylamide compounds such as N-(meth)acryloylmorpholine, N-(meth)acryloylpyrrolidine, N-(meth)acryloylpiperidine, N-(meth)acryloyl-2-methylpiperidine, and N-(meth)acryloyl-2,2,6,6-tetramethylpiperidine.

[Photopolymerization Initiator (C)]

The photopolymerization initiator (C) used in the present invention is used with the monofunctional methacrylic polymerizable compound (A) and the polyfunctional (meth)acrylic polymerizable compound (B) to impart curability in a resin composition for stereolithography of the present invention. The photopolymerization initiator (C) used in the present invention may be selected from polymerization initiators used in industry, preferably photopolymerization initiators used in dentistry.

Examples of the photopolymerization initiator (C) include (bis)acylphosphine oxides, thioxanthones or quaternary ammonium salts of thioxanthones, ketals, α-diketones, coumarins, anthraquinones, benzoin alkyl ether compounds, and α-aminoketone compounds. The photopolymerization initiator (C) may be used alone, or two or more thereof may be used in combination.

Preferably, the photopolymerization initiator (C) of the present invention comprises a photopolymerization initiator with a molecular weight of 400 or more (C)-1 (hereinafter, also referred to simply as "photopolymerization initiator (C)-1"). With a molecular weight of 400 or more, it is possible to reduce leachables from the cured product of a resin composition for stereolithography of the present invention.

Preferred among such photopolymerization initiators (C)-1 is at least one selected from the group consisting of bisacylphosphine oxides and oxime esters. More preferred are bisacylphosphine oxides. In this way, a resin composition for stereolithography can be obtained that has excellent photocurability both in the ultraviolet and visible regions, and that shows sufficient photocurability regardless of whether the light source used is a laser, a halogen lamp, a light emitting diode (LED), or a xenon lamp.

Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide. Other examples include the compounds mentioned in JP2000-159621 A. In view of the leachability of the shaped article, preferred among these are bis(2,6-dichlorobenzoyl)phenylphosphine oxide, bis(2,6-dichlorobenzoyl)-2,5-dimethylphenylphosphine oxide, bis(2,6-dimethoxybenzoyl)phenylphosphine oxide, bis(2,6-dimethoxybenzoyl)-2,5-dimethylphenylphosphine oxide, and bis(2,4,6-trimethylbenzoyl)phenylphosphine oxide, more preferably bis(2,6-dimethoxybenzoyl) phenylphosphine oxide, bis(2,6-dimethoxybenzoyl)-2,5-dimethylphenylphosphine oxide, and bis(2,4,6-trimethylbenzoyl)phenylphosphine oxide, even more preferably bis(2,4,6-trimethylbenzoyl)phenylphosphine oxide.

Examples of oxime esters that can be used as the photopolymerization initiator include 1,2-octanedione 1-[4-(phenylthio)phenyl]-2-(o-benzoyloxime) (another name: 1-[4-(phenylthio)phenyl]octane-1,2-dione-2-(O-benzoyloxime)), and ethanone, 1-[9-ethyl-6-(2-methylbenzoyl)-9H-carbazol-3-yl]-, 1-(o-acetyloxime) (another name: Irgacure OXE-02).

The content of the photopolymerization initiator (C) in a resin composition for stereolithography of the present invention is not particularly limited. However, in view of curability and other properties of the resin composition for stereolithography obtained, the content of photopolymerization initiator (C) is preferably 0.01 to 20 parts by mass relative to total 100 parts by mass of the polymerizable compounds.

When the content of photopolymerization initiator (C) is less than 0.01 parts by mass, polymerization may not sufficiently proceed to form a shaped product. The content of photopolymerization initiator (C) is more preferably 0.05 parts or more by mass, even more preferably 0.1 parts or more by mass, particularly preferably 0.5 parts or more by mass relative to total 100 parts by mass of the polymerizable compounds.

If the content of photopolymerization initiator (C) exceeds 20 parts by mass, it may lead to leaching from the shaped article of the resin composition for stereolithography. The content of photopolymerization initiator (C) is more preferably 15 parts or less by mass, even more preferably 10 parts or less by mass, particularly preferably 5.0 parts or less by mass relative to total 100 parts by mass of the polymerizable compounds.

In view of achieving even superior shapeability by stereolithography while maintaining excellent strength, toughness, water resistance, and stress retention in the shaped article, the content of photopolymerization initiator (C) is preferably 0.5 to 20 parts by mass, more preferably 0.75 to 10 parts by mass, even more preferably 1.0 to 5.0 parts by mass, particularly preferably 1.25 to 2.5 parts by mass relative to total 100 parts by mass of the polymerizable compounds.

In view of the leaching from the cured product of the resin composition for stereolithography obtained, the content of the photopolymerization initiator (C)-1 in the photopolymerization initiator (C) within a resin composition for stereolithography of the present invention is preferably 50 to 100 mass% relative to the total amount of the photopolymerization initiator (C). When the content of photopolymerization initiator (C)-1 is less than 50 mass%, it may lead to increased leachables from the cured product. The content of the photopolymerization initiator (C)-1 is more preferably 75 mass% or more, even more preferably 90 mass% or more, or may be 100 mass%, relative to the total amount of the photopolymerization initiator (C).

A resin composition for stereolithography of the present invention is not particularly limited, and may comprise other components for example, as long as it comprises the monofunctional methacrylic polymerizable compound (A), polyfunctional (meth)acrylic polymerizable compound (B), and photopolymerization initiator (C). The method of production of a resin composition for stereolithography of the present invention is not particularly limited, and a resin composition for stereolithography of the present invention can be produced following known methods of producing resin compositions for stereolithography.

A resin composition for stereolithography of the present invention may comprise a polymerization accelerator to improve photocurability, provided that it does not hinder the intent and purpose of the present invention. Examples of the polymerization accelerator include ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of imparting superior curability to the resin composition for stereolithography, preferred for use is at least one selected from the group consisting of ethyl 4-(N,N-dimethylamino)benzoate, n-butoxyethyl 4-(N,N-dimethylamino)benzoate, and 4-(N,N-dimethylamino)benzophenone.

In a resin composition for stereolithography of the present invention, a filler may be additionally incorporated to adjust paste properties or to improve the mechanical strength of the shaped article of the resin composition for stereolithography. Examples of the filler include organic fillers, inorganic fillers, and organic-inorganic composite fillers. The filler may be used alone, or two or more thereof may be used in combination.

Examples of organic filler materials include polymethyl methacrylate, polyethyl methacrylate, a methyl methacrylate-ethyl methacrylate copolymer, crosslinked polymethyl methacrylate, crosslinked polyethyl methacrylate, polyesters, polyamides, polycarbonates, polyphenylene ethers, polyoxymethylene, polyvinyl chloride, polystyrene, polyethylene, polypropylene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used in combination. There is no particular restriction on the shape of organic filler, and the particle diameter of the filler may be appropriately selected for use.

Examples of inorganic filler materials include quartz, silica, alumina, silica-titania, silica-titania-barium oxide, silica-zirconia, silica-alumina, lanthanum glass, borosilicate glass, soda glass, barium glass, strontium glass, glass-ceramic, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, and strontium calcium fluoroaluminosilicate glass. These may be used alone, or two or more thereof may be used in combination. There is no particular restriction on the shape of inorganic filler, and the inorganic filler may be appropriately selected from inorganic fillers of different shapes, such as irregularly shaped fillers and spherical fillers.

The content of inorganic fillers is not particularly limited, provided that it does not hinder the intent and purpose of the present invention. However, for considerations such as possible embrittlement of dental materials such as denture base materials and dental occlusal splints, or materials for treating sleep disorder, the content of inorganic fillers is preferably 50 parts or less by mass, more preferably 25 parts or less by mass, even more preferably 10 parts or less by mass relative to total 100 parts by mass of the polymerizable compounds.

The content of inorganic fillers is preferably 10 mass% or less, more preferably less than 5 mass%, even more preferably less than 1 mass% in total 100 mass% of the resin composition for stereolithography.

A resin composition for stereolithography of the present invention may comprise a polymer to alter properties such as flexibility and flowability, provided that it does not hinder the intent and purpose of the present invention. Examples include natural rubber, synthetic polyisoprene rubber, liquid polyisoprene rubber and hydrogenated products thereof, polybutadiene rubber, liquid polybutadiene rubber and hydrogenated products thereof, styrene-butadiene rubber, chloroprene rubber, ethylene-propylene rubber, acryl rubber, isoprene-isobutylene rubber, acrylonitrile-butadiene rubber,

and styrene elastomers. Specific examples of other polymers that may be added include a polystyrene-polyisoprene-polystyrene block copolymer, a polystyrene-polybutadiene-polystyrene block copolymer, a poly($\alpha$-methylstyrene)-polybutadiene-poly($\alpha$-methylstyrene) block copolymer, a poly(p-methylstyrene)-polybutadiene-poly(p-methylstyrene) block copolymer, and hydrogenated products of these.

A resin composition for stereolithography of the present invention may optionally comprise a softener. Examples of the softener include petroleum-based softeners such as paraffinic, naphthenic, and aromatic process oils, and vegetable oil-based softeners such as paraffin, peanut oil, and rosin. These softeners may be used alone, or two or more thereof may be used in combination. The softener content is not particularly limited, provided that it does not hinder the intent and purpose of the present invention. Typically, the softener content is 200 parts or less by mass, preferably 100 parts or less by mass relative to total 100 parts by mass of the polymerizable compounds.

A resin composition for stereolithography of the present invention may comprise a known stabilizer, in order to inhibit deterioration or adjust photocurability. Examples of such stabilizers include polymerization inhibitors, ultraviolet absorbers, and antioxidants. These may be used alone, or two or more thereof may be used in combination.

Examples of the polymerization inhibitors include hydroquinone, hydroquinone monomethyl ether, dibutylhydroquinone, dibutylhydroquinone monomethyl ether, 4-t-butyl catechol, 2-t-butyl-4,6-dimethylphenol, 2,6-di-t-butylphenol, and 3,5-di-t-butyl-4-hydroxytoluene. The content of the polymerization inhibitors is preferably 0.001 to 5.0 parts by mass relative to total 100 parts by mass of the polymerizable compounds.

A resin composition for stereolithography of the present invention may comprise a known additive, in order to adjust color tones or paste properties. Examples of such additives include pigments, dyes, organic solvents, and thickeners. These may be used alone, or two or more thereof may be used in combination.

A resin composition for stereolithography of the present invention may additionally comprise polymerizable compounds other than the monofunctional methacrylic polymerizable compound (A) and the polyfunctional (meth)acrylic polymerizable compound (B).

Examples of such additional polymerizable compounds include (meth)acrylamide oligomers (for example, those with a weight-average molecular weight of 1,000 or less, or those with a weight-average molecular weight of 1,000 or more).

In a certain preferred embodiment, a resin composition for stereolithography of the present invention is preferably essentially free of (meth)acrylamide oligomers (for example, those with a molecular weight of 1,000 or less, or those with a molecular weight of 1,000 or more).

Another certain preferred embodiment is, for example, a resin composition for stereolithography that is essentially free of additional polymerizable compounds.

Here, by "essentially free of (meth)acrylamide oligomers", it means that the content of (meth)acrylamide oligomers is less than 5 mass%, preferably less than 1 mass%, more preferably less than 0.1 mass%, even more preferably less than 0.01 mass% in total 100 mass% of the resin composition for stereolithography.

Concerning the content of additional polymerizable compounds, the phrase "essentially free of additional polymerizable compounds" is intended to have the same meaning as the phrase "essentially free of (meth)acrylamide oligomers."

A resin composition for stereolithography of the present invention exhibits excellent strength, toughness, water resistance, and stress retention in the shaped article while reducing leachables, in addition to possessing shapeability. This makes a resin composition for stereolithography of the present invention usable in applications where such advantages can be exploited, including intraoral applications. Examples of intraoral applications include dental materials such as denture base materials and dental occlusal splints; and materials for treating sleep disorder (particularly, appliances for the treatment of sleep apnea). A resin composition for stereolithography of the present invention is particularly suited for denture base materials, dental occlusal splints, and appliances for the treatment of sleep apnea.

A shaped article using a resin composition for stereolithography of the present invention may have a shape that depends on intended use. In a resin composition for stereolithography of the present invention, the type and content of monofunctional methacrylic polymerizable compound (A), polyfunctional (meth)acrylic polymerizable compound (B), and photopolymerization initiator (C), as well as optional components (such as polymerization accelerators, fillers, polymers, softeners, stabilizers, and additives) may be optionally adjusted for different uses, for example, denture base materials, occlusal splints, and appliances for the treatment of sleep apnea.

A resin composition for stereolithography of the present invention can be used in a wide variety of applications by taking advantage of its properties, specifically, the superior dimensional accuracy due to the low rate of volume shrinkage upon curing with light, and the ability to produce shaped products, three-dimensional shaped articles, or other shaped articles that exhibit superior strength, toughness, water resistance, and stress retention in cured form. For example, a resin composition for stereolithography of the present invention can be used for stereolithographical production of three-dimensional shaped articles, and production of various shaped products, for example, film-shaped articles or molded articles produced by a technique such as film casting or casting, and molds for coating and vacuum molding applications.

A resin composition for stereolithography of the present invention is particularly suited for stereolithography such

as above. In stereolithography applications, a resin composition for stereolithography of the present invention enables smooth production of a three-dimensional shaped article having superior toughness and mechanical characteristics while ensuring superior dimensional accuracy with a maintained low rate of volume shrinkage at the time of curing with light.

In view of comfort and usability during use as a denture base material or a dental occlusal splint, a resin composition for stereolithography of the present invention has a flexural modulus ranging preferably from 200 to 3,000 MPa, more preferably from 400 to 2,500 MPa, even more preferably from 600 to 2,000 MPa in cured form.

A certain preferred embodiment is, for example, a resin composition for stereolithography having a flexural modulus of 200 MPa or more and less than 1,900 MPa in cured form.

A resin composition for stereolithography of the present invention has a flexural strength of preferably 15 to 120 MPa, more preferably 20 to 100 MPa, even more preferably 25 to 90 MPa in cured form.

The methods of measurement of flexural modulus and flexural strength are as described in the EXAMPLES below.

Another embodiment of the present invention is a method for producing a three-dimensional shaped article by a stereolithography method using any of the resin compositions for stereolithography described above. The stereolithography method is not particularly limited, and may employ vat photopolymerization techniques such as laser SLA (Stereolithography Apparatus), and DLP (digital light processing) SLA. An example of applicable SLA techniques is LFS (Low Force Stereolithography).

In stereolithography using a resin composition for stereolithography of the present invention, any known stereolithography method and device (for example, a stereolithography device such as the DIGITALWAX® 028J-Plus manufactured by DWS) may be used. In the present invention, the light energy used to cure the resin is preferably an active energy beam. As used herein, "active energy beam" means an energy ray capable of curing a resin composition for stereolithography, and includes, for example, ultraviolet light, an electron beam, X-rays, radiant rays, and high-frequency waves. For example, the active energy beam may be ultraviolet light of 300 to 400 nm wavelengths. The light source of active energy beam may be, for example, a laser such as an Ar laser or a He-Cd laser; or a lighting such as a halogen lamp, a xenon lamp, a metal halide lamp, an LED, a mercury lamp, or a fluorescent lamp. Lasers are particularly preferred. When the light source is a laser, the fabrication time can be reduced by increasing the energy level, and a three-dimensional shaped article of high shape accuracy can be obtained by taking advantage of the desirable convergence of a laser beam.

Stereolithography using a resin composition for stereolithography of the present invention may employ any known method and any known stereolithography system, and the method and device are not particularly limited, as noted above. However, a typical example of a stereolithography method preferred for use in the present invention is a method that produces the desired final three-dimensional shaped article through a repeated procedure that includes a step of forming a cured layer by selectively applying an active energy beam to the resin composition for stereolithography so as to obtain a cured layer having a desired pattern, and a step of continuously forming another cured layer on the previously formed cured layer by similarly applying an active energy beam to a newly supplied, uncured liquid of the resin composition for stereolithography. The resulting three-dimensional shaped article may be used as is, or, depending on the application, it may be used after improving mechanical characteristics, shape stability, or other properties by, for example, post-curing the product under applied light or heat.

The three-dimensional shaped article obtained by stereolithography is not limited to a particular structure, shape, or size, and these may be decided according to use. Typical examples of areas to which the stereolithography method of the present invention is applicable include production of various models and molds, including, for example, models for assessing external designs in a designing process; models for checking functions of components or parts; resin molds for making casting molds; base models for making molds; and direct molds for prototype molds. More specifically, the stereolithography method of the present invention is applicable to, for example, production of models or work models for precision components and parts, electrical/electronic components, furniture, architectural structures, automobile parts, various containers and vessels, castings, molds, and masters. By taking advantage of the excellent strength and toughness of the shaped article of the resin composition for stereolithography, the stereolithography method of the present invention can be very effectively used for, for example, complex-shape cushioning materials in structures (for example, architectural structures), and molds for vacuum molding.

## EXAMPLES

The following describes the present invention in greater detail by way of Examples. It should be noted, however, that the present invention is in no way limited by the following Examples, and various changes may be made by a person with ordinary skill in the art within the technical idea of the present invention.

The components used for the resin compositions for stereolithography according to Examples and Comparative Examples are described below, along with the abbreviations.

[Monofunctional Methacrylic Polymerizable Compound (A)]

POBMA: m-phenoxybenzyl methacrylate (manufactured by Kyoeisha Chemical Co., Ltd.)
EPPMA: ethoxylated o-phenylphenol methacrylate (manufactured by Shin-Nakamura Chemical Co., Ltd.)

[Polyfunctional (Meth)Acrylic Polymerizable Compound with a Molecular Weight of Less Than 500 and Containing Urethane Bond (B)-I-1]

UDMA: 2,2,4-trimethylhexamethylenebis(2-carbamoyloxyethyl)dimethacrylate (manufactured by Kyoeisha Chemical Co., Ltd.; molecular weight: 471)

[Polyfunctional (Meth)Acrylic Polymerizable Compound with a Molecular Weight of Less Than 500 and Containing no Urethane Bond (B)-I-2]

D2.6E: 2,2-bis(4-methacryloyloxypolyethoxyphenyl)propane (with an average of 2.6 moles of ethoxy group added; BPE-100 manufactured by Shin-Nakamura Chemical Co., Ltd.; molecular weight: approximately 478)

[Polyfunctional (Meth)Acrylic Polymerizable Compound with a Molecular Weight of 500 or More and Containing Urethane Bond (B)-II-1]

<Synthesis Example 1> [Production of Polyfunctional Urethanized (Meth)Acrylic Polymerizable Compound UA1]

(1) A 5 L four-neck flask equipped with a stirrer, a thermostat, a thermometer, and a condenser was charged with 250 g of isophorone diisocyanate and 0.15 g of di-n-butyltin dilaurate, and the mixture was heated to 70°C while being stirred.
(2) Separately, 2,500 g of polyester polyol (Kuraray Polyol® P-2050 manufactured by Kuraray Co., Ltd.; a polyol composed of sebacic acid and 3-methyl-1,5-pentanediol; weight-average molecular weight Mw: 2,000) was added into a dropping funnel equipped with a side tube, and the solution in the dropping funnel was dropped into the flask of (1). Here, the solution was dropped at a constant rate over a time period of 4 hours with the temperature inside the flask held at 65 to 75°C while stirring the solution in the flask of (1). After dropping, the mixture was stirred at the same temperature for 2 hours to allow reaction.
(3) Thereafter, a homogenous solution prepared by adding 150 g of 2-hydroxyethyl acrylate and 0.4 g of hydroquinone monomethyl ether into a different dropping funnel was dropped into the flask of (2) at a constant rate over a time period of 2 hours with the temperature inside the flask held at 55 to 65°C, and a reaction was allowed for 4 hours at the maintained solution temperature of 70 to 80°C in the flask to obtain a urethanized (meth)acrylic polymerizable compound UA1. The polyfunctional urethanized (meth)acrylic polymerizable compound UA1 had a weight-average molecular weight Mw of 2,600 as measured by GPC analysis.

[Monofunctional (Meth)Acrylic Polymerizable Compounds Other Than Monofunctional Methacrylic Polymerizable Compound (A)]

POBA: m-phenoxybenzyl acrylate (manufactured by Kyoeisha Chemical Co., Ltd.)
EPPA: ethoxylated o-phenylphenol acrylate (manufactured by Shin-Nakamura Chemical Co., Ltd.)

The weight-average molecular weight Mw of each compound synthesized above means a weight-average molecular weight in terms of polystyrene equivalents, as determined by gel permeation chromatography (GPC).

UA2: polyester-based urethane acrylate oligomer lacking a polyol backbone with a branched structure (UA-210FMD manufactured by Shin-Nakamura Chemical Co., Ltd.; molecular weight: approximately 750)
UA3: N,N'-(2,2,4-trimethylhexamethylene)bis[2-(aminocarboxy)propane-1,3-diol]tetramethacrylate (manufactured by Kyoeisha Chemical Co., Ltd.; molecular weight: 673)

[Polyfunctional (Meth)Acrylic Polymerizable Compound with a Molecular Weight of 500 or More and Containing no Urethane Bond (B)-II-2]

Bis-GMA: 2,2-bis[4-(2-hydroxy-3-methacryloyloxypropoxy)phenyl]propane (manufactured by Shin-Nakamura Chemical Co., Ltd.; molecular weight: 513)

[Photopolymerization Initiator (C)]

BAPO: bis(2,4,6-trimethylbenzoyl)phenylphosphine oxide (Omnirad 819 manufactured by IGM Resins B.V.; molecular weight: 418)
OX1: 1,2-octanedione 1-[4-(phenylthio)phenyl]-2-(o-benzoyloxime) (Irgacure OXE-01 manufactured by BASF Japan; molecular weight: 445)
TPO: 2,4,6-trimethylbenzoyldiphenylphosphine oxide (molecular weight: 348)

[Polymerization Inhibitor]

BHT: 3,5-di-t-butyl-4-hydroxytoluene

[Examples 1 to 14 and Comparative Examples 1 to 5]

The components were mixed at an ordinary temperature (20°C ± 15°C; JIS (Japanese Industrial Standards) Z 8703:1983) in the amounts (parts by mass) shown in Tables 1 and 2 to prepare pastes representing the resin compositions for stereolithography according to Examples 1 to 14 and Comparative Examples 1 to 5.

<Strength (Flexural Modulus, Flexural Strength) and Toughness (Displacement of Flexural Fracture Point)>

The resin composition for stereolithography produced was fabricated into a test specimen with the dimensions (64.0 mm in length, 10.0 mm in width, and 3.3 mm in thickness) described in JIS T 6501:2012 (Acrylic Denture Base Resins), using a stereolithography device (DIGITALWAX® 020D manufactured by DWS) at a pitch of 100 μm with the light exposure time set to 1.3 seconds per layer. A flexure test was conducted using this specimen as a shaped article of the resin composition for stereolithography.

The shaped articles of the resin compositions for stereolithography according to Examples and Comparative Examples were stored in air for 1 day, and measured for strength (flexural modulus, flexural strength) and toughness (displacement of flexural fracture point). The measurements were conducted using a universal testing machine (Autograph AG-I 100kN, manufactured by Shimadzu Corporation) at a crosshead speed of 5 mm/min (n = 5). The arithmetic mean values of the measurements are presented in Tables 1 and 2.

In view of comfort and usability during use as a denture base material or a dental occlusal splint, the preferred flexural modulus range is 200 to 3,000 MPa, more preferably 400 to 2,500 MPa, even more preferably 600 to 2,000 MPa.

The preferred flexural strength is 15 to 120 MPa, more preferably 20 to 100 MPa, even more preferably 25 to 90 MPa.

As for the displacement of fracture point, it is preferable to have no fracture. Specimens were rated "S" (excellent toughness) when there was no fracture, "A" (favorable toughness) when fracture occurred at a displacement of 15 mm or more, "B" (moderate toughness) when fracture occurred at a displacement of 10 mm or more, and "C" (poor toughness) when fracture occurred at a displacement of less than 10 mm, with ratings "S", "A", and "B" considered acceptable.

<Water Resistance>

The resin composition for stereolithography produced was fabricated into a test specimen (64.0 mm in length, 10.0 mm in width, 3.3 mm in thickness), following the same method employed in the flexure test. For the measurement of water resistance, a flexure test was conducted using this specimen as a shaped article of the resin composition for stereolithography.

The shaped articles of the resin compositions for stereolithography according to Examples and Comparative Examples were immersed in 37°C water for 168 hours, and measured for flexural strength in the same manner as in the flexural strength test above (n = 5). The arithmetic mean values of the measurements are presented in Tables 1 and 2. Assuming that the measured flexural strength in the toughness evaluation above is the initial flexural strength, a rate of change (percentage decrease) in flexural strength of 10% or less indicates excellent water resistance, as indicated below.

In Tables 1 and 2, the flexural strength after immersion in 37°C water for 168 hours is denoted as "Flexural strength after immersion."

Rate of change (percentage decrease) in flexural strength (%) = [{initial flexural strength (MPa) - flexural strength after immersion in 37°C water for 168 hours (MPa)} / initial flexural strength (MPa)] × 100

<Stress Retention>

The resin composition for stereolithography produced was fabricated into a specimen (60.0 mm in length, 20.0 mm in width, 1.0 mm in thickness), using a stereolithography device (DIGITALWAX® 020D manufactured by DWS). From this sheet, a test specimen was prepared in the same dimensions specified for the dumbbell-shaped No. 8 specimen in JIS K 6251:2010 (Rubber, vulcanized or thermoplastic-Determination of tensile stress-strain properties), using a punching blade. A tensile stress relaxation test was then conducted using this specimen as a shaped article of the resin composition for stereolithography.

Specifically, in a 25°C environment, the dumbbell-shaped specimen was secured by holding the both ends with tensile test chucks mounted in a tensile testing apparatus (EZ Test EZ-SX manufactured by Shimadzu Corporation), with the chucks spaced 10 mm apart. The distance between the chucks was then increased by 1.0 mm to apply a 10% displacement, and the maximum stress observed was designated as the initial stress. While maintaining the 10% displacement, the test specimen was held still for 24 hours, and the stress after this 24-hour period was observed (n = 1). Stress relaxation is expressed by the equation below. Stress retention was considered favorable when the stress relaxation was 70% or less, and particularly favorable when the stress relaxation was 60% or less.

Stress relaxation rate (%) = [{initial tensile stress (MPa) - tensile stress (MPa) after 24 hours} / initial tensile stress (MPa)] × 100

<Leachability>

The resin composition for stereolithography produced was fabricated into a disc (15 mm in diameter, 1.0 mm in thickness), using a stereolithography device (DIGITALWAX® 020D manufactured by DWS) at a pitch of 50 μm with the light exposure time set to 0.6 seconds per layer. A single disc was then immersed in 2.0 ml of ethanol in a 10 ml glass vial at 50°C for 72 hours. After immersion, the disc was removed, and the ethanol was distilled away before measuring the mass of the resulting extract (n = 1).

An amount of leachables less than or equal to 1.0 mg was evaluated as indicating a low level of leachables.

[Table 1]

| Components (parts by mass) | | | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 | Ex. 14 |
|---|---|---|---|---|---|---|---|---|---|---|---|---|---|---|---|---|---|
| Raw materials | | POBMA | | 20 | | 20 | 20 | 20 | 20 | 20 | 10 | 35 | 20 | 20 | 20 | 40 | 40 |
| | | EPPMA | | | 20 | | | | | | | | | | | | |
| | (B)-I D2.6E | UDMA | | 45 | 45 | | 45 | 45 | 45 | 45 | 60 | 40 | 35 | 40 | 20 | 60 | |
| | | D2.6E | | | | 45 | | | | | | | | 5 | 25 | | |
| | (B)-II | UA1 | | 35 | 35 | 35 | | | | 35 | 30 | 25 | 45 | 30 | 15 | | |
| | | UA2 | | | | | 35 | | | | | | | | | | 60 |
| | | UA3 | | | | | | 35 | | | | | | | | | |
| | | Bis-GMA | | | | | | | 35 | | | | | | 20 | | |
| | (C)-1 | BAPO | | 1.5 | 1.5 | 1.5 | 1.5 | 1.5 | 1.0 | | 1.5 | 2.0 | 2.0 | 1.5 | 1.5 | 1.5 | 1.5 |
| | | OX1 | | | | | | | | 1.5 | | | | | | | |
| | | BHT | | 0.5 | 0.5 | 0.5 | 0.5 | 0.5 | 0.1 | 0.5 | 0.5 | 0.5 | 1.0 | 0.5 | 0.5 | 0.5 | 0.5 |
| Properties | Strength | Flexural strength | (MPa) | 28 | 29 | 30 | 85 | 92 | 91 | 27 | 34 | 27 | 24 | 30 | 56 | 90 | 64 |
| | | Flexural modulus | (MPa) | 720 | 780 | 820 | 1600 | 1800 | 1900 | 690 | 860 | 710 | 580 | 830 | 1200 | 1800 | 1400 |
| | Toughness | Displacement of fracture point | | S | S | A | A | B | B | S | S | S | S | S | A | B | A |
| | Water resistance | Flexural strength after immersion | (MPa) | 27 | 27 | 29 | 82 | 84 | 82 | 25 | 33 | 26 | 22 | 28 | 51 | 82 | 58 |
| | | Percentage decrease | (%) | 3.6 | 6.9 | 6.7 | 3.5 | 8.7 | 9.9 | 7.4 | 2.9 | 3.7 | 8.3 | 6.7 | 8.9 | 8.9 | 9.4 |
| | Stress retention | Stress relaxation rate | (%) | 55 | 58 | 67 | 56 | 54 | 67 | 57 | 58 | 53 | 59 | 58 | 64 | 52 | 68 |
| | Leachability | Amount of leachables | mg | 0.1 | 0.7 | 0.1 | 0.2 | 0.2 | 0.3 | 0.3 | 0.1 | 0.2 | 0.2 | 0.2 | 0.5 | 0.2 | 0.6 |

[Table 2]

| Components (parts by mass) | | | | | Ex. 1 | Com. Ex. 1 | Com. Ex. 2 | Com. Ex. 3 | Com. Ex. 4 | Com. Ex. 5 |
|---|---|---|---|---|---|---|---|---|---|---|
| Raw materials | (A) | POBMA | | | 20 | | | 100 | | |
| | | EPPMA | | | | | | | | |
| | (B)-I | UDMA | | | 45 | 45 | 45 | | 60 | 45 |
| | | D2.6E | | | | | | | | |
| | (B)-II | UA1 | | | 35 | 35 | 35 | | 40 | 35 |
| | | UA2 | | | | | | | | |
| | | UA3 | | | | | | | | |
| | | Bis-GMA | | | | | | | | |
| | | POBA | | | | 20 | | | | 20 |
| | | EPPA | | | | | 20 | | | |
| | (C)-1 | BAPO | | | 1.5 | 1.5 | 1.5 | 1.5 | 1.5 | |
| | | OX1 | | | | | | | | |
| | (C) | TPO | | | | | | | | 1.5 |
| | | BHT | | | 0.5 | 0.5 | 0.5 | 0.5 | 0.5 | 0.5 |
| Properties | Strength | Flexural strength | (MPa) | | 28 | 27 | 28 | 12 | Not shapable due to high viscosity | 29 |
| | | Flexural modulus | (MPa) | | 720 | 700 | 750 | 180 | | 740 |
| | Toughness | Displacement of fracture point | | | S | S | S | C | | S |
| | Water resistance | Flexural strength after immersion | (MPa) | | 27 | 26 | 27 | 11 | | 28 |
| | | Percentage decrease | (%) | | 3.6 | 3.7 | 3.6 | 8.3 | | 3.4 |
| | Stress retention | Stress relaxation rate | (%) | | 55 | 78 | 74 | 92 | | 72 |
| | Leachability | Amount of leachables | mg | | 0.1 | 0.2 | 0.8 | 10.4 | | 3.2 |

As indicated in Tables 1 and 2, the shaped articles of the resin compositions for stereolithography of Examples 1 to 14, through the integrated action of the components, exhibited excellent strength, toughness, water resistance, and stress retention with reduced amounts of leachables. In particular, the shaped articles of the resin compositions for stereolithography according to Examples 1 to 14 were superior to the resin composition of Comparative Example 3 in terms of the strength and toughness of the shaped articles.

The shaped articles of the resin compositions for stereolithography according to Examples 1 to 14 exhibited superior stress retention compared to the resin compositions of Comparative Examples 1 to 3, and 5.

The shaped articles of the resin compositions for stereolithography according to Examples 1 to 14 released fewer leachables than the shaped articles of the resin compositions of Comparative Examples 3 and 5.

By contrasting Example 1 with Comparative Example 1 and Comparative Example 2, it was confirmed that the resin composition for stereolithography cannot achieve sufficient stress retention with a combination of components containing monofunctional acrylic polymerizable compounds without monofunctional methacrylic polymerizable compound (A).

By contrasting Example 1 with Comparative Example 3, it was confirmed that sufficient strength, toughness, and

stress retention cannot be achieved with a combination of components lacking the polyfunctional (meth)acrylic polymerizable compound (B).

By contrasting Example 1 with Comparative Example 4, it was confirmed that a combination of components lacking the monofunctional methacrylic polymerizable compound (A) in the resin composition for stereolithography leads to high viscosity and prevents shaping.

INDUSTRIAL APPLICABILITY

A resin composition for stereolithography of the present invention exhibits excellent strength, toughness, water resistance, and stress retention in the shaped article while reducing leachables from the shaped article. This makes a resin composition for stereolithography of the present invention suited for intraoral applications, such as a variety of dental materials (particularly, denture base materials and dental occlusal splints) or various materials for treating sleep disorder (particularly, appliances for the treatment of sleep apnea).

In particular, because a resin composition for stereolithography of the present invention can reduce a decrease in stress while maintaining strength and toughness, it can be prevented from losing its tooth corrective capability when used as an orthodontic aligner.

**Claims**

**1.** A resin composition for stereolithography, comprising a monofunctional methacrylic polymerizable compound having a plurality of independent aromatic rings (A), a polyfunctional (meth)acrylic polymerizable compound (B), and a photopolymerization initiator (C).

**2.** The resin composition for stereolithography according to claim 1, wherein the monofunctional methacrylic polymerizable compound having a plurality of independent aromatic rings (A) has two aromatic rings.

**3.** The resin composition for stereolithography according to claim 1 or 2, wherein the monofunctional methacrylic polymerizable compound having a plurality of independent aromatic rings (A) comprises at least one selected from the group consisting of o-phenoxybenzyl methacrylate and m-phenoxybenzyl methacrylate.

**4.** The resin composition for stereolithography according to claim 1 or 2, wherein the polyfunctional (meth)acrylic polymerizable compound (B) comprises a polyfunctional (meth)acrylic polymerizable compound with a molecular weight of less than 500 (B)-I.

**5.** The resin composition for stereolithography according to claim 1 or 2, wherein the polyfunctional (meth)acrylic polymerizable compound (B) comprises a polyfunctional (meth)acrylic polymerizable compound with a molecular weight of 500 or more (B)-II.

**6.** The resin composition for stereolithography according to claim 1 or 2, wherein the polyfunctional (meth)acrylic polymerizable compound (B) comprises a polyfunctional (meth)acrylic polymerizable compound with a molecular weight of less than 500 (B)-I, and a polyfunctional (meth)acrylic polymerizable compound with a molecular weight of 500 or more (B)-II.

**7.** The resin composition for stereolithography according to claim 4, wherein the polyfunctional (meth)acrylic polymerizable compound (B) comprises a polyfunctional (meth)acrylic polymerizable compound with a molecular weight of less than 500 and containing a urethane bond (B)-I-1, and/or a (meth)acrylic polymerizable compound with a molecular weight of 500 or more and containing a urethane bond (B)-II-1.

**8.** The resin composition for stereolithography according to claim 4, wherein the polyfunctional (meth)acrylic polymerizable compound (B) comprises a polyfunctional (meth)acrylic polymerizable compound with a molecular weight of less than 500 and containing a urethane bond (B)-I-1, and a (meth)acrylic polymerizable compound with a molecular weight of 500 or more and containing a urethane bond (B)-II-1.

**9.** The resin composition for stereolithography according to claim 7, which comprises the polyfunctional (meth)acrylic polymerizable compound (B)-I-1, wherein the polyfunctional (meth)acrylic polymerizable compound (B)-I-1 comprises a (meth)acrylate that comprises no polymer structure within a single molecule.

**10.** The resin composition for stereolithography according to claim 7, which comprises the (meth)acrylic polymerizable compound (B)-II-1, wherein the (meth)acrylic polymerizable compound (B)-II-1 comprises a (meth)acrylate that comprises, within a single molecule, at least one structure selected from the group consisting of a polyester, a polycarbonate, a polyurethane, a polyether, a poly-conjugated diene, and a hydrogenated poly-conjugated diene.

**11.** The resin composition for stereolithography according to claim 10, wherein the (meth)acrylic polymerizable compound (B)-II-1 comprises a (meth)acrylate that comprises, within a single molecule, at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic chain diol unit having a branched structure.

**12.** The resin composition for stereolithography according to claim 1 or 2, wherein the photopolymerization initiator (C) comprises a photopolymerization initiator with a molecular weight of 400 or more (C)-1.

**13.** The resin composition for stereolithography according to claim 12, wherein the photopolymerization initiator with a molecular weight of 400 or more (C)-1 comprises bis(2,4,6-trimethylbenzoyl)phenylphosphine oxide.

**14.** A dental material comprising a shaped article of the resin composition for stereolithography of claim 1 or 2.

**15.** A denture base material comprising a shaped article of the resin composition for stereolithography of claim 1 or 2.

**16.** A dental occlusal splint comprising a shaped article of the resin composition for stereolithography of claim 1 or 2.

**17.** A material for treating sleep disorder comprising a shaped article of the resin composition for stereolithography of claim 1 or 2.

**18.** A method for stereolithographically producing a three-dimensional shaped article using the resin composition for stereolithography of claim 1 or 2.

## INTERNATIONAL SEARCH REPORT

International application No. **PCT/JP2024/037281**

**A. CLASSIFICATION OF SUBJECT MATTER**

***C08F 220/10***(2006.01)i; ***A61C 7/08***(2006.01)i; ***A61C 13/10***(2006.01)i; ***A61K 6/15***(2020.01)i; ***A61K 6/60***(2020.01)i; ***A61K 6/62***(2020.01)i; ***A61M 16/06***(2006.01)i; ***B29C 64/124***(2017.01)i; ***B29C 64/314***(2017.01)i; ***B33Y 80/00***(2015.01)i; ***C08F 2/44***(2006.01)i; ***C08F 290/00***(2006.01)i

FI: C08F220/10; A61C7/08; A61C13/10; A61K6/15; A61K6/60; A61K6/62; A61M16/06 D; B29C64/124; B29C64/314; B33Y80/00; C08F2/44 B; C08F290/00

According to International Patent Classification (IPC) or to both national classification and IPC

**B. FIELDS SEARCHED**

Minimum documentation searched (classification system followed by classification symbols)

C08F220/10; A61C7/08; A61C13/10; A61K6/15; A61K6/60; A61K6/62; A61M16/06; B29C64/124; B29C64/314; B33Y80/00; C08F2/44; C08F290/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2021-088150 A (KURARAY NORITAKE DENTAL INC.) 10 June 2021 (2021-06-10) claims, example 6, paragraph [0011] | 1-3, 5, 7, 10-18 |
| Y | | 4, 6, 8-9 |
| X | WO 2020/246610 A1 (KURARAY NORITAKE DENTAL INC.) 10 December 2020 (2020-12-10) claims, example 6, paragraph [0012] | 1-3, 5, 7, 10-18 |
| Y | | 4, 6, 8-9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 November 2024** | **17 December 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | Telephone No. |

Form PCT/ISA/210 (second sheet) (July 2022)

**INTERNATIONAL SEARCH REPORT**

International application No.
**PCT/JP2024/037281**

**C. DOCUMENTS CONSIDERED TO BE RELEVANT**

| Category* | Citation of document, with indication, where appropriate, of the relevant passages | Relevant to claim No. |
|---|---|---|
| X | WO 2023/191112 A1 (KURARAY NORITAKE DENTAL INC.) 05 October 2023 (2023-10-05)<br>claims, examples 1-5, 7-9, paragraph [0186] | 1-4, 7, 9, 14-15 |
| Y | | 5-6, 8, 10-11, 18 |
| A | | 12-13, 16-17 |
| X | WO 2023/120611 A1 (KURARAY NORITAKE DENTAL INC.) 29 June 2023 (2023-06-29)<br>claims, examples 1-2, 4-5, paragraphs [0006]-[0007] | 1-4, 7, 9, 14 |
| Y | | 5-6, 8, 10-11 |
| A | | 12-13, 15, 16-18 |
| Y | WO 2023/190931 A1 (KURARAY NORITAKE DENTAL INC.) 05 October 2023 (2023-10-05)<br>paragraphs [0001]-[0010], [0019], [0022], [0029], [0034], [0047], [0106], [0121] | 4-6, 8-11, 18 |
| A | | 1-3, 7, 12-17 |

Form PCT/ISA/210 (second sheet) (July 2022)

**INTERNATIONAL SEARCH REPORT**
**Information on patent family members**

International application No.
**PCT/JP2024/037281**

| Patent document cited in search report | Publication date (day/month/year) | Patent family member(s) | Publication date (day/month/year) |
|---|---|---|---|
| JP 2021-088150 A | 10 June 2021 | (Family: none) | |
| WO 2020/246610 A1 | 10 December 2020 | US 2022/0259361 A1<br>claims, example 6, paragraph [0014]<br>EP 3981810 A1<br>CN 113906068 A | |
| WO 2023/191112 A1 | 05 October 2023 | (Family: none) | |
| WO 2023/120611 A1 | 29 June 2023 | CN 118414136 A | |
| WO 2023/190931 A1 | 05 October 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020246610 A
- JP 2021088150 A
- WO 2019189566 A
- JP 2020158417 A
- WO 2021162007 A
- JP 2000159621 A